# EUROPÄISCHE PATENTSCHRIFT

(11) **EP 0 965 435 B1**
(45) Veröffentlichungstag und Bekanntmachung des Hinweises auf die Patenterteilung: **23.03.2005**
(21) Anmeldenummer: 99106988.1
(22) Anmeldetag: 09.04.1999
(51) Int. Cl.: B29C 51/14, B29C 51/10, A61L 27/44, B29C 70/08

(54) **Verfahren zur Herstellung von partiell verstärkten Kunststoffbauteilen**
Method for manufacturing partly reinforced plastic parts
Procédé pour la fabrication des composants en matière plastique renforcés partièllement

(30) Priorität: 20.06.1998 DE 19827550
(43) Veröffentlichungstag der Anmeldung: 22.12.1999
(73) Patentinhaber: Otto Bock HealthCare GmbH, 37115 Duderstadt (DE)
(72) Erfinder: Deinert, Jürgen, 37115 Duderstadt (DE); Segl, Maximilian, 37434 Gieboldehausen (DE); Bendlin, Regina, 37075 Göttingen (DE)
(74) Vertreter: Lins, Edgar, Dipl.-Phys. Dr.jur.

(56) Entgegenhaltungen:
- EP-A- 0 195 562
- DE-A- 1 491 209
- GB-A- 1 363 305
- US-A- 4 230 764
- US-A- 5 219 364
- US-A- 5 571 355
- PATENT ABSTRACTS OF JAPAN vol. 009, no. 245 (M-418), 2. Oktober 1985 (1985-10-02) & JP 60 097826 A (FUJI JUKOGYO KK;OTHERS: 01), 31. Mai 1985 (1985-05-31)
- PATENT ABSTRACTS OF JAPAN vol. 005, no. 006 (M-050), 16. Januar 1981 (1981-01-16) & JP 55 139215 A (MITSUBISHI RAYON CO LTD;OTHERS: 01), 30. Oktober 1980 (1980-10-30)

## Beschreibung

Die Erfindung betrifft ein Verfahren zur Herstellung von thermoplastischen, partiell verstärkten Kunststoffbauteilen durch Warmformung.

Gebräuchlich ist z. B. ein mechanisches Verbinden oder Verkleben von warmgeformten Bauteilen mit einer metallischen Verstärkung. Nachteilig hierbei sind das hohe spezifische Gewicht der Metalle sowie die hohen Fertigungskosten.

Eine partielle Verstärkung von durch Warmformung hergestellten Bauteilen wird im Stand der Technik auch durch Einbringen von Sicken erreicht. Hierdurch lässt sich jedoch nur ein sehr begrenzter Verstärkungseffekt realisieren.

Es ist ferner bekannt, das Verstärkungsmaterial als Einlegeteil in eine Spritzguss- oder Pressform zu legen, anschließend die Press- oder Spritzgussmasse in die Form einzubringen und so das Bauteil auszuformen. Als Werkstoff für die Einlegeteile kommen überwiegend Metalle aber auch faserverstärkte Kunststoffe zum Einsatz. Diese Verfahren eignen sich jedoch in erster Linie für Großserien, sind aber aufgrund der hohen Werkzeugkosten für Bauteile mit kleineren Produktionsstückzahlen unwirtschaftlich.

Aus US 5,219,364 ist ein Verfahren zur Herstellung von thermoplastischen, eventuell partiell verstärkten, Kunststoffbauteilen durch Warmformung bekannt, wobei eine zuvor plastifizierte Kunststoffplatte auf einer Positiv-Tiefziehform durch Anlegen von Vakuum geformt wird.

Durch GB 1 363 305 sind orthopädische Implantatbauteile bekannt, die aus einem faserverstärkten Silikonkunststoff bestehen. Diese Teile können mit einer Außenschicht umhüllt, also mehrlagig aufgebaut sein. Die Herstellung erfolgt in üblicher Spritztechnik, wobei die verwendeten Graphitfasern trocken in die Form eingelegt und der Kunststoff anschließend in die Form eingespritzt werden kann, um dann in der Form auszuhärten.

Beim Warmformen von thermoplastimprägnierten Geweben (Organobleche) wird die Wirtschaftlichkeit des Verfahrens vom hohen Materialpreis kompensiert. Außerdem ist mit derartigen Materialien lediglich eine vollständige, nicht aber eine partielle Verstärkung der Bauteile möglich.

Der Erfindung liegt die Aufgabe zugrunde, ein Verfahren zu entwickeln, mit dem sich thermoplastische, partiell verstärkte Kunststoffbauteile auch bei kleinen Produktionsstückzahlen mit geringen Fertigungskosten herstellen lassen.

Diese Aufgabe wird erfindungsgemäß dadurch gelöst, dass zur Bildung der partiell verstärkten Bauteilbereiche warmformbare Verstärkungsmaterialien verwendet werden, die Fasern mit einer Lange > 1 mm enthalten, durch Wärmeübertragung in weichelastischen Zustand überführt und dann auf eine Positiv-Tiefziehform an definierten Stellen und in definierter Orientierung drapiert werden, worauf dann eine zuvor plastifizierte Kunststoffplatte über die mit dem Verstärkungsmaterial drapierte Form gezogen und durch Anlegen von Vakuum an die Form unter Einwirkung des Atmosphärendruckes so gegen die Formoberfläche und das Verstärkungsmaterial gedrückt wird, dass die vorhandene Wärmemenge des Verstärkungsmaterials zu dessen Verschweißung mit der Kunststoffplatte führt.

Das warmformbare Verstärkungsmaterial muss in den weichelastischen Zustand überführt werden, um die Kontur der Form nachbilden zu können. Da die Struktur des Verstärkungsmaterials (Geometrie, Faserlänge, Faserorientierung) erhalten bleiben soll, kann lediglich eine Wärmeübertragung, nicht aber eine Scherung oder dergleichen eingesetzt werden. Als Wärmequellen können z. B. Infrarotstrahler, Wärmeschrank oder strömende Heißluft verwendet werden.

Bei der Herstellung von Bauteilen mit einfacher Geometrie kann als Verstärkungsmaterial ein thermoplastimprägnierter Roving eingesetzt werden. Da das Verstärkungsmaterial im Gegensatz zur Spritzgieß- oder Preßverarbeitung keiner Nachkonsolidierung unterzogen werden kann, können nur Materialien mit 100%-igem Imprägniergrad eingesetzt werden. Materialien mit geringerem Imprägniergrad versagen bei dynamischer Beanspruchung aufgrund der Reibung der Fasern aufeinander.

Für Bauteile mit komplexer Geometrie und dadurch bedingt zeitaufwendiger Drapierung des Verstärkungsmaterials an der Form sind einfache thermoplastimprägnierte Rovings aufgrund ihrer geringen Wärmekapazität nicht geeignet.

Um die Schweißverbindung zwischen Verstärkungsmaterial und Kunststoffplatte sicherzustellen, muß das Verstärkungsmaterial eine ausreichende Wärmemenge aufweisen. Bei zeitaufwendiger Drapierung der Form sinkt jedoch die Temperatur des in weichelastischen Zustand überführten Verstärkungsmaterials unter die erforderliche Verarbeitungstemperatur. Dabei liegt der Zeitbedarf zum Drapieren der Verstärkungsmaterialen auf der Form je nach Geometrie und Anzahl der benötigten Verstärkungsmaterialien zwischen etwa 10 und 120 Sekunden. Weist dann das Verstärkungsmaterial eine zu geringe Wärmemenge und/oder zu hohe Wärmeleitfähigkeit auf, muß die plastifizierte Kunststoffplatte eine ausreichende Wärmemenge auf das Verstärkungsmaterial übertragen. Dabei kann davon ausgegangen werden, daß diese Wärmeabgabe nur von einer etwa 1 mm starken Grenzschicht der Kunststoffplatte erfolgen kann.

Zur Erhöhung der Wärmekapazität des Verstärkungsmaterials kann auch dessen Schichtstärke heraufgesetzt werden. Hierfür ist es zweckmäßig, wenn als Verstärkungsmaterial ein Schichtverbundwerkstoff verwendet wird, der zumindest eine faserhaltige Verstärkungsschicht und eine als Wärmespeicher dienende Schicht aus unverstärktem Kunststoff aufweist. Ein derartiger Schichtverbund erweist sich im Vergleich zu homogenen Verstärkungsmaterialien hinsichtlich Gewicht und Isolationswirkung insbesondere gegenüber einer kalten Form als vorteilhaft.

Die Fasern können im Verstärkungsmaterial unidirektional ausgerichtet sein; in diesem Fall wird die Verstärkungswirkung der Fasern voll ausgenutzt. Bei einer multidirektionalen Verteilung der Fasern im Verstärkungsmaterial ist der Verstärkungseffekt geringer ausgeprägt; vorteilhaft hierbei ist jedoch eine erhöhte Sicherheit gegen Delamination.

Alle Varianten des Verstärkungsmaterials erlauben problemlos eine Individualfertigung partiell verstärkter Kunststoffbauteile. Hierdurch wird eine prinzipiell neue Konzeption von Bauteilen ermöglicht, die z. B. als Prothesen oder Orthesen für die individuelle Patientenversorgung Verwendung finden können.

Es lassen sich Verstärkungsmaterialien verarbeiten, deren Fasern eine Länge von mehr als 25 mm aufweisen. Derartige Verstärkungsmaterialien weisen eine hohe Dauerschwingfestigkeit sowie eine durch Variation von Faserart, Faserkonzentration und Schichtstärke einstellbare Biegesteifigkeit auf. Nach dem erfindungsgemäßen Verfahren hergestellte Kunststoffbauteile lassen sich daher auch als Federelemente in Bauteile integrieren (z. B. als Federelement in einer Dynamik-Fußprothese).

Ferner sind Systeme denkbar, in denen das Verstärkungsmaterial unter definierter Krafteinleitung einen derartigen Biegewinkel zuläßt, daß eine Gelenkfunktion gegeben ist. Gleichzeitig wirkt das Verstärkungsmaterial dann als Federelement, wodurch eine Rückholfunktion gegeben ist, sobald die zur Biegung notwendige Kraft nicht mehr auf das Bauteil einwirkt.

Zur Vorbereitung der erfindungsgemäß zu verarbeitenden Kunststoffplatte ist es zweckmäßig, wenn die zu plastifizierende Kunststoffplatte in einen Rahmen eingespannt und solange erwärmt wird, bis sie einen weichelastischen Zustand erreicht hat, im Rahmen durchhängt und somit vorgestreckt wird. Es ist aber auch möglich, die plastifizierte Kunststoffplatte durch Druckluft vorzustrecken.

Die Positiv-Tiefziehform kann aus Metall, Holz, wärmeformbeständigem Kunststoff bzw. Kunststoffschaum, Gips oder dergleichen bestehen. Die Oberfläche muß geschlossen und glatt sein, um Formschluß zwischen der Form und dem erstarrenden Thermoplast zu vermeiden. Bei Formen aus Gips und ähnlich porösen Materialien ist es zweckmäßig, wenn die Oberfläche der Positiv-Tiefziehform beschichtet oder mit einem wärmeformbeständigen Gewebe oder einer entsprechenden Folie überzogen wird. Hierbei ist darauf zu achten, daß das Gewebe oder die Beschichtung keine Schweißverbindung mit dem Thermoplasten eingehen kann und faltenfrei aufgebracht wird.

Bei Kleinserien und Individualfertigung bzw. bei entsprechend langen Zykluszeiten ist es nicht erforderlich, die Positiv-Tiefziehform auf eine bestimmte Temperatur einzustellen. Jedoch ist es vorteilhaft, wenn insbesondere bei Serienfertigung und dadurch bedingter kurzer Zykluszeit die Formtemperatur geregelt wird.

Das erfindungsgemäße Verfahren kann wie folgt durchgeführt werden:

Zuerst wird das Verstärkungsmaterial erwärmt, bis es sich im weichelastischen Zustand befindet. Anschließend wird das Verstärkungsmaterial an den gewünschten Stellen auf die Positiv-Tiefziehform drapiert. Gleichzeitig wird eine Kunststoffplatte in einen Rahmen eingespannt und solange erwärmt, bis sie sich im weichelastischen Zustand befindet. Aufgrund ihres Eigengewichts wird sie ab einer bestimmten Temperatur durchhängen und somit vorgestreckt. Ist die gewünschte Temperatur bzw. der gewünschte Vorstreckgrad erreicht, wird der Rahmen mit der Kunststoffplatte auf die Form und die darauf befindlichen Verstärkungsmaterialien abgesenkt. Durch Anlegen von Vakuum wird dann der weichelastische Kunststoff unter Einwirkung des Atmosphärendrucks gegen die Formoberfläche sowie gegen das Verstärkungsmaterial gepreßt und dadurch das Formteil ausgebildet. Das Verstärkungsmaterial muß sich zu diesem Zeitpunkt noch im weichelastischen Zustand befinden, oder aber die Kunststoffplatte muß eine ausreichende Wärmemenge zur Erweichung des Verstärkungsmaterials übertragen können. Unter diesen Bedingungen stellt sich dann eine Verschweißung zwischen Kunststoffplatte und Verstärkungsmaterial ein.

Ein wesentlicher Vorteil des erfindungsgemäßen Verfahrens ist darin zu sehen, daß sich Verstärkungen nur an den Positionen in ein Tiefzieh-Bauteil einbringen lassen, wo es für die Funktion vorteilhaft ist, während an anderen Stellen im Bauteil höhere Flexibilität zugelassen werden kann.

Der für die genannte Verschweißung erforderliche Wärmehaushalt läßt sich beispielsweise unter folgenden Prozeßparametern realisieren:
a) Positiv-Tiefziehform:
   Verwendet wird ein untemperiertes Gipsmodell, das mit Nylongewebe überzogen ist.
   Verstärkungsmaterial:
   - Zusammensetzung: 60 Gew.-% Glasfaser unidirektional, 40 Gew.-% Polypropylen
   - Abmessung: 0,05 x 0,5 x 20 cm = 1 cm³
   - therm. Eigenschaft: Wärmekapazität Cp = 1,54 J/g K Wärmeleitfähigkeit λ = 0,42 W/m K
   - Temperatur: 25°C (kühlt nach dem Drapieren auf Raumtemperatur ab!)
   - benötigte Wärmemenge: 160 J (Erwärmen von 25°C auf 165°C) pro Verstärkungsstreifen.
   Kunststoffplatte:
   - Zusammensetzung: 100 % Polypropylen
   - Volumen Grenzschicht: 0,1 x 0,5 x 20 cm = 2 cm³
   - therm. Eigenschaft: Wärmekapazität Cp = 1,9 J/g K Wärmeleitfähigkeit λ =0,17 W/m K
   - Temperatur: 250°C
   - übertragbare Wärmemenge: 270 J (Abkühlen von 250°C auf 170°C) pro Verstärkungsstreifen.
b) Ein gänzlich anderer Wärmehaushalt wird realisiert, wenn das Verstärkungsmaterial eine derart hohe Wärmemenge aufweist, daß das Material nicht unterhalb der zur Verschweißung notwendigen Temperatur abkühlt. Solche Verhältnisse liegen vor, wenn das Verstärkungsmaterial ausreichend Masse und Temperatur aufweist. Die Wärmeübertragung vom Verstärkungsmaterial zur Form kann durch eine hohe Wandstärke sowie durch eine niedrige Wärmeleitfähigkeit des Verstärkungsmaterials minimiert werden.
c) Unter folgenden Prozeßparametern konnte ein Abkühlen des aus einem Schichtverbund bestehenden Verstärkungsmaterials unterhalb der Verschweißungstemperatur vermieden werden:
   Form: untemperiertes Gipsmodell, mit übergezogenem Nylongewebe
   Verstärkungsmaterial:
   - Zusammensetzung Verstärkungsschicht: 60 Gew.-% Glasfaser unidirektional, 40 Gew.-% Polypropylen
   - Abmessung Verstärkungsschicht: 0,05 x 2,5 x 20 cm = 2,5 cm³ (= 3,73 g)
   - Zusammensetzung Wärmespeicher- und Isolationsschicht: 100 % Polypropylen
   - Abmessung Wärmespeicher- und Isolationsschicht: 0,1 x 2,5 x 20 cm = 5,0 cm³ (= 4,5 g)
   - Therm. Eigenschaft: Wärmekapazität Cp = 1,74 J/g K Wärmeleitfähigkeit λ =0,25 W/m K
   - Temperatur: 250°C (kühlt nach dem Drapieren auf T > 170°C ab!)
   - maximal zulässiger Wärmeverlust: 1120 J pro Verstärkungsstreifen
   Es hat sich gezeigt, dass die Wärmemenge von 1120 J nicht innerhalb von 120 s aus dem Verstärkungsmaterial abgegeben werden kann. Daher ist eine Verschweißung sichergestellt.

Das erwärmte und somit weichelastische Verstärkungsmaterial neigt zur Haftung auf der Positiv-Tiefziehform. Daher genügt zur Befestigung der Verstärkungsmaterialien auf der Form in vielen Fällen ein einfaches Auflegen. An senkrechten Flächen kann das Verstärkungsmaterial mit kleinen Stiften befestigt werden.

Das erfindungsgemäße Verfahren erlaubt die Herstellung eines neuen thermoplatischen, partiell verstärkten, an seiner Innenseite an einer Positivform warmgeformten Kunststoffbauteils mit einer durch Warmformung geformten Platte und einem an die Innenseite der Platte angesetzten Verstärkungsteil, das aus einem mit Fasern mit einer Länge > 1 mm enthaltenden Verstärkungsmaterial besteht, mit seiner Innenseite an der Positivform geformt und mit seiner Außenseite mit der Platte verschweißt ist, die durch Anlegen an der Positivform zusammen mit dem geformten Verstärkungsteil geformt ist.

Das erfindungsgemäß hergestellte Kunststoffbauteil ist zuverlässig partiell verstärkt und, wie besprochen, einfach auch in Einzelanfertigung herstellbar.

Bevorzugt werden erfindungsgemäß hergestellte Kunststoffbauteile als Federelement, als Gelenkersatz mit einem Feder-Rückholsystem oder Teile einer Prothese oder Orthese verwendet.

In der Zeichnung sind einige als Beispiele dienende Ausführungsformen der Erfindung schematisch dargestellt. Es zeigen:
Figur 1: die einzelnen Verfahrensschritte zur Herstellung einer Fußorthese;
Figur 2: einzelne Verfahrensschritte zur Herstellung eines Knöchelgelenks als Gelenk mit Feder-Rückholsystem und
Figur 3: ein erfindungsgemäß hergestelltes Kunststoffbauteil in Form eines Chéneau-Boston-Wiesbaden-Korsetts.

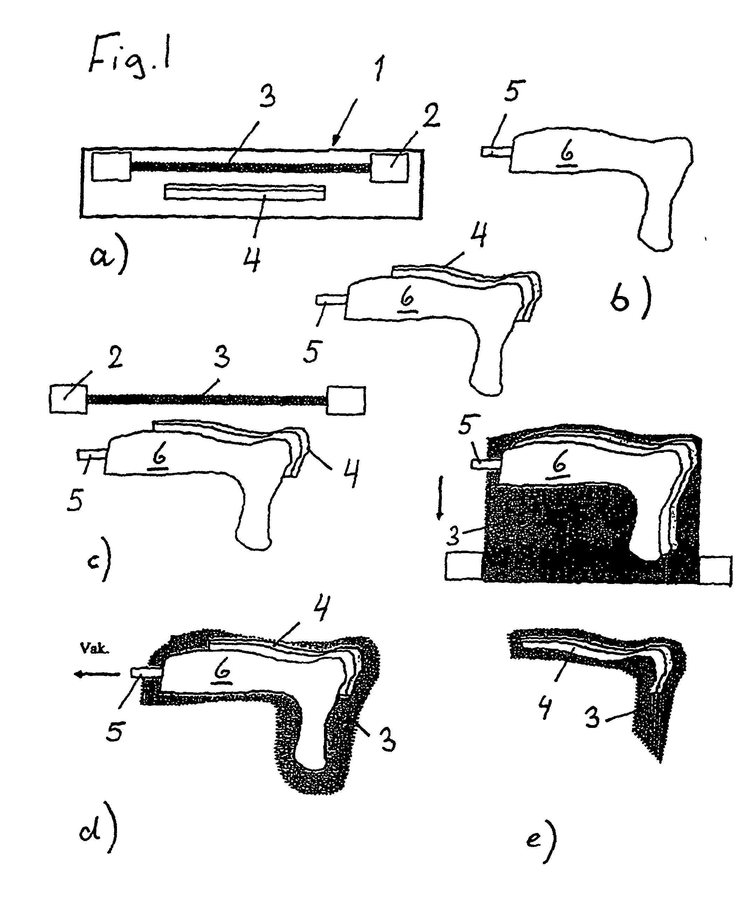

In Figur 1 ist unter a) schematisch ein Wärmeofen 1 angedeutet, in dem eine in einem Spannrahmen 2 montierte Polypropylenplatte 3 zusammen mit einem aus Polypropylen/Glasfaser-Verstärkungsschicht und Polypropylen-Wärmespeicher/Isolationsschicht bestehenden Verstärkungsmaterial 4 auf 250°C erwärmt werden. Anschließend wird das Verstärkungsmaterial 4 auf eine mit Vakuumanschluss 5 versehene Gipsform 6 eines Patientenfußes drapiert (1b). Nach spätestens 120 s wird die Polypropylenplatte 3 über die Positiv-Tiefziehform 6 gezogen (1c). Durch Anlegen von Vakuum wird das Plattenmaterial gegen die Form 6 sowie gegen das Verstärkungsmaterial 4 gepreßt (1d). Nach Abkühlen kann das Bauteil entformt und spanend nachgearbeitet werden (1e).

Bei dieser Herstellung der Fußorthese können folgende Bedingungen eingestellt werden:
Form: Gipsmodell, mit Nylongewebe überzogen, Raumtemperatur (untemperiert)
   Verstärkungsmaterial:
   Schichtverbundwerkstoff
   - 0,05 x 2,5 x 20 cm, 60 Gew.-% Glasfaser unidirektional, 40 Gew.-% Polypropylen mit
   - 0,3 x 2,5 x 20 cm, 100 Gew.-% Polypropylen als Wärmespeicher- und Isolationsschicht
   - Vorwärmtemperatur: 250°C
   Kunststoffplatte:
   - Zusammensetzung: 100% Polypropylen
   - Abmessung: 0,3 x 70 x 50 cm
   - Vorwärmtemperatur: 250°C

In Figur 2 ist schematisch die Herstellung eines Knöchelgelenks als Gelenk mit Feder-Rückholsystem nach dem erfindungsgemäßen Verfahren dargestellt.

Auf eine mit PTFE beschichtete 2-teilige Aluminiumform 7 werden zwei Verstärkungselemente 8, jeweils bestehend aus 50 Gew.-% unidirektional angeordneten Carbonfasern und 50 Gew.-% PA 12 drapiert. Über die Aluminiumform 7 mit den drapierten, streifenförmigen Verstärkungselementen 8 wird anschließend eine plastifizierte PA 12-Platte 3 gezogen (a). Nach dem Erkalten wird das Gelenk entformt und spanend nachgearbeitet (b). Zum Abschluß werden Rohrverbindungsmuffen 9 eingesetzt (c). Die Abbildung (d) zeigt das fertige Gelenk in gebeugtem Zustand.

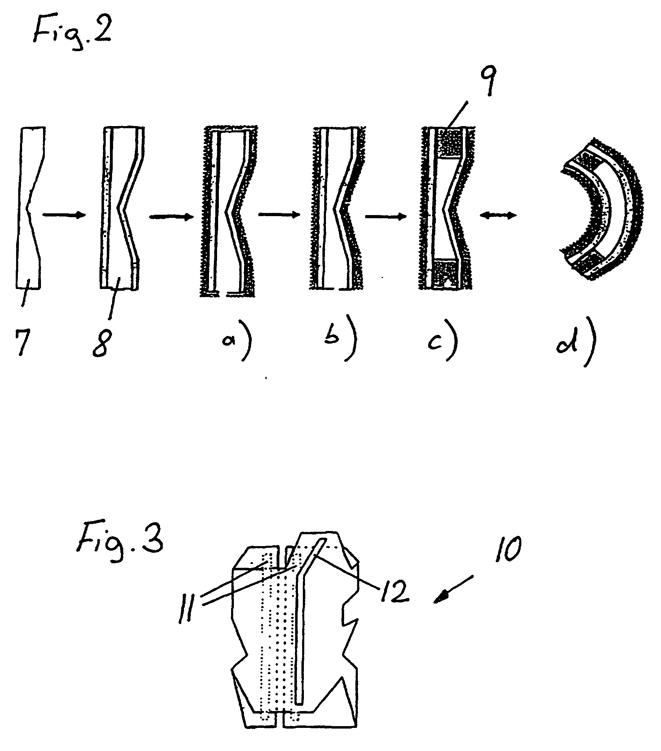

Figur 3 zeigt ein nach dem erfindungsgemäßen Verfahren hergestelltes Chéneau-Boston-Wiesbaden-Korsett 10. Bei diesem Bauteil sind auf seiner Rückseite parallel zu der Rückenöffnung zwei Verstärkungselemente 11 angeordnet. Auf der Vorderseite des Bauteils befindet sich ein weiteres, in Brusthöhe um ca. 40° abgewinkeltes Verstärkungselement 12.

## Patentansprüche

1. Verfahren zur Herstellung von thermoplastischen, partiell verstärkten Kunststoffbauteilen durch Warmformung, **bei dem** zur Bildung der partiell verstärkten Bauteilbereiche warmformbare Verstärkungsmaterialien verwendet werden, die Fasern mit einer Länge > 1 mm enthalten, durch Wärmeübertragung in weichelastischen Zustand überführt und dann auf eine Positiv-Tiefziehform an definierten Stellen und in definierter Orientierung drapiert werden, worauf dann eine zuvor plastifizierte Kunststoffplatte über die mit dem Verstärkungsmaterial drapierte Form gezogen und durch Anlegen von Vakuum an die Form unter Einwirkung des Atmosphärendruckes so gegen die Formoberfläche und das Verstärkungsmaterial gedrückt wird, dass die vorhandene Wärmemenge des Verstärkungsmaterials zu dessen Verschweißung mit der Kunststoffplatte führt.

2. Verfahren nach Anspruch 1, bei dem die zur Erzielung einer Schweißverbindung erforderliche Wärmemenge von der plastifizierten Kunststoffplatte auf das Verstärkungsmaterial übertragen wird.

3. Verfahren nach Anspruch1 oder 2, bei dem die Fasern im Verstärkungsmaterial uni- oder multidirektional angeordnet werden.

4. Verfahren nach einem der vorhergehenden Ansprüche, bei dem die Fasern im Verstärkungsmaterial homogen verteilt werden.

5. Verfahren nach einem der vorhergehenden Ansprüche, bei dem als Verstärkungsmaterial Abschnitte aus mit thermoplastischen Matrices imprägnierten Faserbündeln verwendet werden.

6. Verfahren nach Anspruch 1 oder 2, bei dem als Verstärkungsmaterial ein Schichtverbundwerkstoff verwendet wird, der zumindest eine faserhaltige Verstärkungsschicht und eine als Wärmespeicher dienende Schicht aus unverstärktem Kunststoff aufweist.

7. Verfahren nach einem der vorhergehenden Ansprüche, bei dem die zu plastifizierende Kunststoffplatte in einen Rahmen eingespannt und solange erwärmt wird, bis sie einen weichelastischen Zustand erreicht hat, im Rahmen durchhängt und somit vorgestreckt wird.

8. Verfahren nach einem der Ansprüche 1 bis 6, bei dem die plastifizierte Kunststoffplatte durch Druckluft vorgestreckt wird.

9. Verfahren nach einem der vorhergehenden Ansprüche, bei dem die Oberfläche der Positiv-Tiefziehform beschichtet oder mit einem wärmeformbeständigen Gewebe oder einer entsprechenden Folie überzogen wird.

## Claims

1. Process for producing thermoplastic, partially reinforced plastic components by thermoforming, wherein thermoformable reinforcing materials which contain fibres of a length > 1 mm are used for forming the partially reinforced component regions, transformed into a pliable state by heat transfer and then draped over a positive thermoforming mould at defined points and in a defined orientation, whereupon a previously plasticized plastic sheet is then drawn over the mould draped with the reinforcing material and, by applying a vacuum to the mould, is pressed by the action of atmospheric pressure against the mould surface and the reinforcing material in such a way that the existing amount of heat of the reinforcing material leads to the latter being welded with the plastic sheet.

2. Process according to Claim 1, wherein the amount of heat required for achieving a welded bond is transferred from the plasticized plastic sheet to the reinforcing material.

3. Process according to Claim 1 or 2, wherein the fibres in the reinforcing material are arranged unidirectionally or multidirectionally.

4. Process according to one of the preceding claims, wherein the fibres in the reinforcing material are distributed homogeneously.

5. Process according to one of the preceding claims, wherein portions from fibre strands impregnated with thermoplastic matrices are used as the reinforcing material.

6. Process according to Claim 1 or 2, wherein a laminar material which has at least one fibre-containing reinforcing layer and a layer of unreinforced plastic serving as a heat store is used as the reinforcing material.

7. Process according to one of the preceding claims, wherein the plastic sheet to be plasticized is clamped into a frame and heated until it has reached a pliable state, sags in the frame and is consequently pre-stretched.

8. Process according to one of Claims 1 to 6, wherein the plasticized plastic sheet is pre-stretched by compressed air.

9. Process according to one of the preceding claims, wherein the surface of the positive thermoforming mould is coated or is covered with a heat-resistant woven fabric or a corresponding film.

## Revendications

1. Procédé pour fabriquer des composants en matière plastique partiellement renforcés par formage à chaud, dans lequel on utilise pour réaliser les zones partiellement renforcées des composants, des matériaux de renforcement formables à chaud, comprenant des fibres de longueur > à 1 mm, qui sont soumis à un transfert de chaleur pour obtenir un état mou et élastique et ensuite sont drapés sur une forme positive d'emboutissage profond selon des positions définies et des orientations définies, sur laquelle on étire, sur la forme drapée du matériau de renforcement, une plaque en matière plastique préalablement plastifiée et par l'application d'un vide à la forme et sous l'action de la pression atmosphérique sur la surface supérieure de la forme de façon que le matériau de renforcement soit pressé pour que la quantité de chaleur restante du matériau de renforcement se soude avec la plaque en matière plastique.

2. Procédé selon la revendication 1, dans lequel pour obtenir une liaison par soudure, la quantité de chaleur nécessaire est transférée de la plaque en matière plastique vers le matériau de renforcement.

3. Procédé selon la revendication 1 ou 2, dans lequel les fibres sont disposées dans le matériau de renforcement, de façon uni ou multidirectionnelle.

4. Procédé selon l'une des revendications précédentes, dans lequel les fibres sont distribuées de façon homogène dans le matériau de renforcement.

5. Procédé selon l'une des revendications précédentes, dans lequel on utilise en tant que matériau de renforcement des sections de faisceaux de fibres enrobés dans une matrice thermoplastique.

6. Procédé selon la revendication 1 ou 2, dans lequel on utilise en tant que matériau de renforcement un produit sous forme de couches liées présentant au moins une couche de renforcement comportant des fibres et une couche en matière synthétique non renforcée jouant le rôle d'accumulateur de chaleur.

7. Procédé selon l'une des revendications précédentes, dans lequel pour plastifier la plaque en matière plastique, celle-ci est tendue dans un cadre et est chauffée pendant une durée suffisante pour obtenir un état mou et plastique, qu'elle pende à travers le cadre et soit ainsi étirée.

8. Procédé selon l'une des revendications 1 à 6, dans lequel la plaque en matière plastique plastifiée est étirée par de l'air sous pression.

9. Procédé selon l'une des revendications précédentes, dans lequel la surface supérieure de la forme positive d'emboutissage profond est revêtue ou recouverte par un tissu indéformable à la chaleur ou par une pellicule correspondante.
